# FASCICULE DE BREVET EUROPEEN

(11) **EP 2 131 874 B1**
(45) Date de publication et mention de la délivrance du brevet: **27.08.2014**
(21) Numéro de dépôt: 08787903.7
(22) Date de dépôt: 03.04.2008
(51) Int. Cl.: A61L 2/24, A61B 19/00, G06F 19/00, G06Q 50/22

(54) **PROCEDE DE SUIVI DE L'UTILISATION D'UN APPAREIL MEDICAL**
VERFAHREN ZUR VERFOLGUNG DER VERWENDUNG MEDIZINISCHER GERÄTE
METHOD FOR TRACKING THE USE OF A MEDICAL APPARATUS

(30) Priorité: 05.04.2007 FR 0754299
(43) Date de publication de la demande: 16.12.2009
(73) Titulaire: Germitec, 92110 Clichy (FR)
(72) Inventeur: DESHAYS, Clément, 07120 Ruoms (FR)
(74) Mandataire: Jacobson, Claude
(86) Numéro de dépôt international: PCT/FR2008/000464
(87) Numéro de publication internationale: WO 2009/004126

(56) Documents cités:
- EP-A- 1 402 904
- EP-A1- 1 155 654
- WO-A-99/66444
- WO-A-2005/048041
- DE-A1- 4 420 707
- DE-A1- 19 514 284

## Description

La présente invention concerne un procédé de suivi de l'utilisation d'un appareil médical du type sonde afin de déterminer si cet appareil peut être utilisé sans risque du point de vue hygiénique ainsi qu'un système de suivi permettant de mettre en oeuvre ce procédé.

Dans le domaine médical, l'hygiène des appareils médicaux employés est primordiale afin d'éviter tout risque d'infection d'un patient à cause d'un appareil pas ou mal désinfecté. C'est pourquoi il est indispensable d'assurer une traçabilité de la désinfection afin d'être sûr qu'un appareil a bien été désinfecté avant son utilisation.

Or, actuellement, cette traçabilité est assurée par des opérations manuelles du personnel médical. Ainsi, une fiche accompagne la sonde médicale sur laquelle le personnel médical doit inscrire les opérations de désinfection qui ont été effectuées, ainsi que la façon dont la sonde est utilisée. Dans certains cas, la traçabilité est partiellement automatisée et les informations relatives à une sonde sont regroupées dans une base de données. Cependant, dans ce dernier cas, le personnel médical doit tout de même entrer lui-même les informations relatives à la désinfection et à l'utilisation de la sonde dans la base de données. Par conséquent, la traçabilité est assurée au travers de beaucoup d'interventions humaines qui peuvent être source d'erreur et/ou de falsifications. Il est donc impossible de savoir de façon absolument certaine si une sonde peut être utilisée de façon légitime du point de vue hygiénique et de la lutte contre les infections nosocomiales.

Les documents WO-A-2005/048041 et DE-A-4420707 divulguent des procédés et des systèmes de suivi de l'utilisation d'appareils médicaux.

L'invention a pour but de pallier ces inconvénients en proposant un procédé de suivi de l'utilisation d'une sonde rendant automatique la déduction de la légitimité de l'utilisation de la sonde et limitant l'intervention humaine.

A cet effet, l'invention a pour objet un procédé de suivi de l'utilisation d'un appareil médical du type sonde afin de déterminer si cet appareil peut être utilisé sans risque du point de vue hygiénique, ledit appareil comprenant des moyens d'identification dudit appareil, le procédé utilisant un système d'acquisition et de traitement d'informations comprenant des moyens d'acquisition des informations d'identification dudit appareil, des moyens d'acquisition d'informations relatives à la désinfection et à l'utilisation dudit appareil et des moyens d'association des informations d'identification et d'utilisation dudit appareil, ledit système comprenant un appareil indépendant et portable apte à communiquer avec un système de traitement d'informations, ledit appareil comprenant les moyens d'acquisition des informations d'identification (Iᵢ) de l'appareil médical, les moyens d'acquisition d'informations d'utilisation relatives à l'utilisation dudit appareil médical et les moyens d'horodatage, le procédé comprenant les étapes suivantes :
- lors de la désinfection de l'appareil, enregistrement par le système d'acquisition et de traitement de la date de la désinfection et des informations de désinfection relati-ves au type et au degré de désinfection de l'appareil en relation avec les informations d'identification dudit appareil,
- lors de l'utilisation de l'appareil sur un patient, enregistrement par le système d'acquisition et de traitement de la date et des informations d'utilisation relatives au type d'utilisation de l'appareil et au patient en relation avec les informations d'identification dudit appareil,
- séquençage par le système d'acquisition et de traitement des informations de désinfection et des informations d'utilisation sur un patient en relation avec les informations d'identification de l'appareil,
- préalablement à chaque utilisation, élaboration par le système d'acquisition et de traitement d'une instruction d'autorisation ou d'interdiction de l'utilisation de l'appareil à partir du séquençage, l'utilisation étant autorisée si, suite à une utilisation particulière, une désinfection adaptée à cette utilisation a eu lieu, et l'utilisation étant interdite dans le cas contraire.

Par séquençage, on entend que les informations de désinfection et les informations d'utilisation sont mises les unes à la suite des autres en fonction du moment où elles ont été enregistrées dans le temps de sorte à former un historique des évènements subis par l'appareil médical.

L'invention permet donc d'interdire l'utilisation de l'appareil médical si son utilisation n'est pas légitime du point de vue de l'hygiène, par exemple si l'appareil n'a pas été suffisamment désinfecté ou si l'utilisation a lieu trop longtemps après que l'appareil a été désinfecté. On assure ainsi une traçabilité efficace de l'appareil médical qui ne risque pas d'être utilisé si une certaine chaîne d'événements (étape de désinfection) ne s'est pas produite auparavant.

Selon d'autres caractéristiques du procédé :
- il comprend en outre une étape d'enregistrement d'une information relative au temps de stockage de l'appareil par le système d'acquisition et de traitement, ladite information de stockage étant séquencée avec les informations de désinfection et d'utilisation, le système d'acquisition et de traitement élaborant une instruction de désinfection si le temps de stockage dépasse un seuil prédéterminé et une instruction d'interdiction d'utilisation de l'appareil si cette désinfection n'est pas effectuée,
- l'enregistrement des informations relatives au type et au degré de la désinfection comprend l'enregistrement du principe actif utilisé pour la désinfection, le temps d'exposition à ce principe actif de l'appareil, la température de désinfection et/ou le dosage du principe actif,
- le système d'acquisition et de traitement élabore une information de suffisance ou d'insuffisance de la désinfection en fonction des informations relatives au type et au degré de désinfection, le système élaborant une instruction d'interdiction d'utilisation de l'appareil si l'information d'insuffisance de la désinfection est élaborée,
- au cours de la désinfection de l'appareil, un enregistrement de l'identité de l'opérateur effectuant la désinfection est effectué,
- l'enregistrement des informations des informations relatives au type d'utilisation de l'appareil comprend l'enregistrement de l'identité du médecin traitant, de l'identité du patient, du type de traitement pour lequel l'appareil est utilisé, du type de pathologie du patient, de la durée d'utilisation de l'appareil, des informations sur l'environnement dans lequel est utilisé l'appareil et/ou des informations sur le système dans lequel est utilisé l'appareil,
- le système d'acquisition et de traitement enregistre un historique du suivi de l'utilisation de l'appareil médical et de sa légitimité d'utilisation.

L'invention concerne également un système de suivi de l'utilisation d'un appareil médical du type sonde permettant de mettre en oeuvre le procédé tel que décrit ci-dessus, ledit système étant caractérisé en ce qu'il comprend des moyens d'identification associés à l'appareil médical, un système d'acquisition et de traitement d'informations comprenant des moyens d'acquisition des informations d'identification dudit appareil, des moyens d'acquisition d'informations relatives à l'utilisation dudit appareil, des moyens d'horodatage et des moyens d'association des informations d'identification, d'utilisation dudit appareil et d'horodatage.

Les moyens d'acquisition des informations d'identification, les moyens d'acquisition d'informations d'utilisation relatives à l'utilisation dudit appareil, les moyens d'horodatage sont regroupé au sein d'un appareil indépendant et portable apte à communiquer avec un système de traitement d'informations.

Selon d'autres caractéristiques du système :
- les moyens d'identification associés à l'appareil médical comprennent une puce RFID fixée à demeure sur ledit appareil,
- les moyens d'acquisition des informations d'identification de l'appareil médical du système d'acquisition et de traitement comprennent des moyens de lecture à distance d'une puce RFID,
- il comprend des moyens de mesure du degré de désinfection fourni par une enceinte de désinfection,
- il comprend des moyens d'entrée d'informations relatives à l'utilisation de l'appareil médical,
- il comprend des moyens de stockage des informations acquises et traitées par le système d'acquisition et de traitement.

L'invention sera mieux comprise à la lecture de la description qui va suivre, donnée uniquement à titre d'exemple et faite en se référant au dessin annexé, sur lequel :
- la Fig. 1 est un diagramme représentant les différentes étapes du procédé de suivi selon l'invention,
- la Fig. 2 est un diagramme détaillant l'étape de désinfection du procédé.

L'appareil médical selon l'invention est par exemple une sonde utilisée avec un échographe de façon connue. Une telle sonde ne sera pas décrite en détail ici, mais comprend typiquement une partie active et une partie de connectique à l'échographe. La partie active, en contact avec les patients lors d'un examen médical, doit être impérativement stérilisée avant toute utilisation sur un patient afin d'assurer une hygiène satisfaisante et de lutter contre les infections nosocomiales.

Une traçabilité de la désinfection et de l'utilisation de la sonde est donc requise afin de déterminer si une désinfection adéquate de la partie active de la sonde a bien eu lieu avant l'utilisation de la sonde sur un patient. Afin d'assurer cette traçabilité, la sonde comprend des moyens d'identification fixés à demeure sur la sonde.

Ces moyens d'identification sont propres à chaque sonde. Afin de permettre une automatisation du système de suivi ces moyens d'identification comprennent par exemple une puce RFID moulée dans un boîtier plastique fixé à la sonde ou noyée dans le matériau constituant la sonde. Ces moyens d'identification peuvent être lus à distance par des moyens d'acquisition des informations d'identification de la sonde.

Les moyens d'acquisition comprennent par exemple un lecteur RFID apte à lire les informations contenues sur la puce RFID associée à la sonde. Les informations d'identification peuvent être transmises à une unité centrale D de traitement des données appartenant à un système d'acquisition et de traitement de données permettant d'assurer le suivi de l'utilisation de la sonde, comme cela sera décrit ultérieurement.

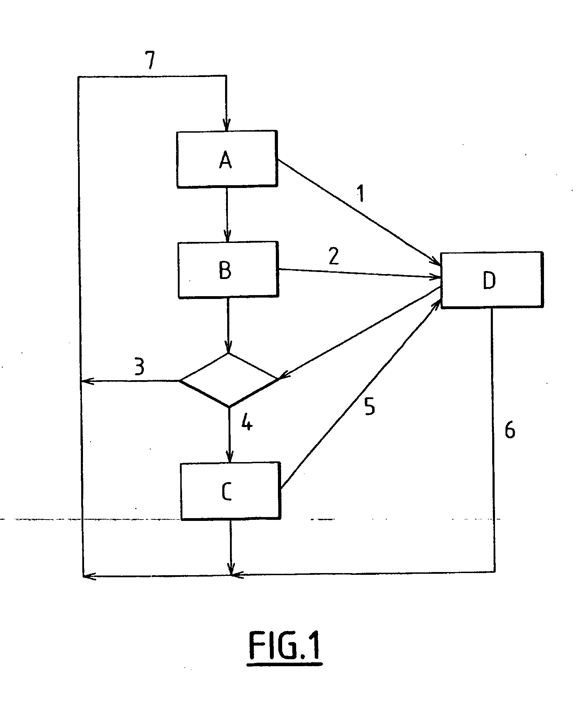

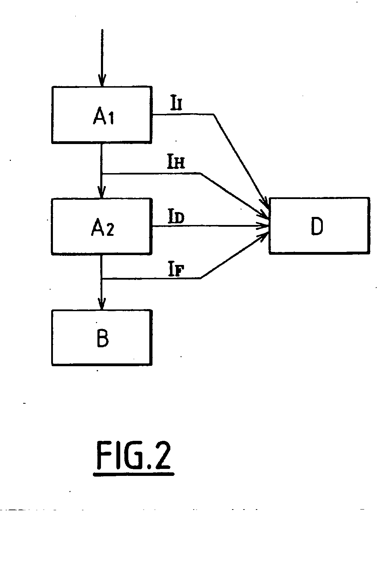

Au cours d'une étape A représentée sur la figure 1, la sonde est désinfectée. Préalablement à cette désinfection, la sonde est identifiée et la date de la désinfection est enregistrée, ainsi que l'heure de début de la désinfection (étape A1 de la figure 2). A la fin de la désinfection, l'heure de fin de la désinfection est enregistrée. Les informations d'identification Iᵢ, les informations de date et de début d'examen Iₕ et les informations de fin de désinfection I_{f} sont transmises à l'unité centrale de traitement des données D, comme représenté par la flèche 1 de la figure 1 et par la figure 2. Un enregistrement de l'identité de l'opérateur effectuant la désinfection est également effectué.

Les moyens d'acquisition des informations d'identification, ainsi que les moyens d'horodatage font partie d'un appareil indépendant et portable disposé dans la salle de désinfection.

Selon un mode de réalisation, l'appareil indépendant et portable est fixé à la sonde, de sorte qu'il suit la sonde dans tous ses déplacements. Un tel appareil indépendant est par exemple une étiquette électronique associée à une mémoire et fixée à la sonde.

Selon un mode de réalisation, on prévoit que l'enceinte de désinfection comporte des moyens d'identification de sorte que l'identité de l'enceinte utilisée pour désinfecter la sonde soit transmise à l'unité de traitement des données D.

La désinfection est réalisée au cours d'une étape A2. La désinfection est par exemple réalisée par l'exposition de la partie active de la sonde à un rayonnement UV, par trempage dans un bain comprenant un principe actif permettant la désinfection, par exposition à un liquide pulvérisé ou par exposition à un gaz comprenant un principe actif permettant la désinfection. Dans tous les cas, des moyens d'acquisition d'informations relatives à l'utilisation dudit appareil permettent d'enregistrer les caractéristiques de la désinfection. Ces moyens d'acquisition comprennent par exemple des moyens de mesure du degré de désinfection fourni par une enceinte de désinfection. Les informations enregistrées comprennent par exemple l'enregistrement du principe actif utilisé pour la désinfection, le temps d'exposition à ce principe actif de l'appareil, la température de désinfection et/ou le dosage du principe actif. Ces mesures peuvent être effectuées au moyen de capteurs ou de tout autre moyens connus. Ces informations de désinfection I_{d} sont transmises à l'unité de traitement des données D (figure 2) qui détermine un degré de désinfection à partir de ces informations. Par exemple, si le dosage du principe actif est mesuré, différents degrés de désinfection seront atteints lorsque le dosage mesuré dépassera des seuils prédéterminés de dosage. Si un rayonnement UV est utilisé pour la désinfection, le temps d'exposition de la sonde à ce rayonnement combiné à la mesure du rayonnement délivré dans l'enceinte permet de déterminer le degré de désinfection.

Ainsi, à l'issue de l'étape A, le système de suivi de l'utilisation d'une sonde connaît le type, le degré et le moment de la désinfection de la sonde dont l'utilisation est suivie.

Selon l'invention un appareil indépendant est prévu, tel qu'un appareil fixé à la sonde comme décrit ci-dessus; un tel appareil regroupe les moyens d'acquisition des informations d'identification Iᵢ, des moyens d'acquisition d'informations d'utilisation relatives à l'utilisation dudit appareil, les moyens d'horodatage. L'appareil est capable de communiquer avec un système de traitement d'informations tel que l'unité de traitement des données D. Un tel appareil est utilisé pour recueillir l'identité de l'opérateur effectuant la désinfection, par exemple au moyen d'un badge introduit dans un moyen de lecture du boîtier et l'identité de la sonde à désinfectée. Une fois, la désinfection effectuée, l'opérateur introduit les informations sur le degré de désinfection ou l'appareil communique avec l'unité de traitement des données D afin d'obtenir les informations sur ce degré de désinfection.

Au cours d'une étape B, la sonde est stockée en attendant son utilisation. Lorsque la sonde est placée dans une enceinte de stockage, elle est identifiée et la date est enregistrée. On enregistre également l'heure du début du stockage. Lorsque la sonde est sortie de l'enceinte de stockage, l'heure de la fin de stockage est enregistrée. Ces informations sont transmises à l'unité de traitement D, comme représenté par la flèche 2 de la figure 1.

A cet effet, l'enceinte de stockage comprend des moyens d'acquisition de l'identité de la sonde du même type que ceux prévus pour l'étape de désinfection. Ces moyens font partie de l'appareil indépendant et portable disposé dans la salle de stockage. Selon une réalisation, l'appareil indépendant utilisé est le même que celui employé pour la désinfection et il suit la sonde dans la salle de stockage. Selon une réalisation, on prévoit que l'enceinte de stockage comporte des moyens d'identification de sorte que l'identité de l'enceinte utilisée pour stocker la sonde soit transmise à l'unité de traitement des données D.

L'unité de traitement des données D croise les informations obtenues sur la désinfection de la sonde avec les informations obtenues sur son stockage. Par croiser les informations, on entend que l'unité de traitement met en relation les informations de désinfection avec les informations de stockage à partir des informations d'identification de la sonde, de sorte que pour une identité de sonde, l'unité de traitement peut par exemple comparer le degré de désinfection de la sonde avec son temps de stockage.

En effet, il correspond un temps de stockage maximal à chaque type de désinfection et de degré de désinfection. Si le temps de stockage de la sonde dépasse le temps de stockage maximal correspondant au type et au degré de désinfection effectué sur la sonde, il est nécessaire d'effectuer une nouvelle désinfection pour s'assurer que la sonde peut être utilisée de façon légitime du point de vue de l'hygiène.

Ainsi, lorsque la sonde est sortie de l'enceinte de stockage pour son utilisation, l'unité de traitement des données D vérifie que la sonde a été désinfectée et compare le temps de stockage de la sonde au temps de stockage maximal correspondant à la désinfection subie par la sonde.

Si la sonde n'a pas été désinfectée avant son stockage, l'unité de traitement des données D élabore une instruction d'interdiction d'utilisation de la sonde et l'enceinte de sockage émet un signal de refus de la sonde. La sonde n'est alors pas utilisée et est renvoyée à l'étape A de désinfection, comme représenté par la flèche 3 de la figure 1.

Si le temps de stockage dépasse le temps de stockage maximal, l'unité de traitement des données élabore une instruction d'interdiction d'utilisation de la sonde. La sonde n'est alors pas utilisée et est renvoyée à l'étape A de désinfection, comme représenté par la flèche 3 de la figure 1.

Si le temps de stockage est inférieur au temps de stockage maximal, la sonde peut être utilisée sans risque du point de vue de l'hygiène. L'unité de traitement des données D élabore alors une instruction d'autorisation d'utilisation de la sonde. La sonde peut alors être utilisée au cours d'une étape C d'utilisation, comme représenté par la flèche 4 de la figure 1.

A l'étape C d'utilisation de la sonde sur un patient, la sonde est identifiée et la date de l'examen est enregistrée. Cette identification et la date sont transmises à l'unité de traitement des données D, comme représenté par la flèche 5 de la figure 1. L'unité de traitement croise ces informations et un séquençage entre la désinfection et l'utilisation est réalisé. Ainsi pour une identité de sonde donnée, toutes les informations relatives aux opérations précédant l'utilisation sont connues et mises en ordre chronologique. On s'assure ainsi qu'une chaîne d'opération a bien été menée avant de permettre l'utilisation de la sonde. Les opérations de vérification de l'identité de la sonde et de la légitimité d'utilisation sont réalisées par l'appareil indépendant et portable déjà utilisé au cours de la désinfection et du stockage. L'appareil indépendant comprend un moyen de lecture d'un badge d'identification du médecin.

Ainsi, si la sonde identifiée n'a pas été désinfectée préalablement à son utilisation, l'unité de traitement des données D élabore une instruction d'interdiction d'utilisation de la sonde et la communique à l'appareil d'imagerie ou à l'appareil indépendant et portable. La sonde n'est alors pas utilisée et est renvoyée à l'étape A de désinfection, comme représenté par la flèche 6 de la figure 1.

Si une instruction d'autorisation d'utilisation de la sonde est élaborée et que l'opérateur entend effectivement utiliser la sonde, il confirme cette utilisation. Cette confirmation est enregistrée par le système de suivi de l'utilisation de la sonde, ce qui permet de déterminer clairement si une sonde a été utilisée ou non. Une telle confirmation de l'utilisation est primordiale, en particulier si plusieurs sondes sont reliées à un même appareil d'imagerie médicale. En effet, dans ce cas, plusieurs sondes peuvent être activées sans pour autant être utilisées par la suite et il devient impossible de voir depuis l'extérieur si une sonde a été effectivement utilisée ou non. La confirmation de l'utilisation de la sonde permet d'identifier avec certitude quelle sonde a été effectivement utilisée et quelles sondes ont simplement été activées sans être utilisées par la suite. La confirmation de l'utilisation permet de déterminer avec certitude les besoins en désinfection d'une sonde particulière.

Le système de suivi de l'utilisation de la sonde comprend des moyens d'entrée d'informations relatives à l'utilisation de l'appareil médical. Ainsi, le médecin qui utilise la sonde sur un patient peut entrer des informations sur l'utilisation de la sonde pour fournir des données à l'unité de traitement des données D. Les informations entrées par le médecin comprennent par exemple l'identité du médecin, le nom du patient, le type de traitement pour lequel la sonde est utilisée, la type de pathologie du patient et/ou des informations sur le système (type d'échographe par exemple) dans lequel est utilisé l'appareil. En pratique, ces informations peuvent également être récupérées dans l'unité de traitement des données D par l'appareil indépendant.

Un enregistrement de la durée d'utilisation de la sonde et des informations sur l'environnement dans lequel est utilisée la sonde peut également être prévu grâce à des moyens similaires (capteurs, etc.) à ceux employés pour la désinfection de la sonde.

La confirmation de l'utilisation d'une sonde ainsi que toutes les informations mentionnées ci-dessus sont communiquées à l'unité de traitement de données D qui les croise avec les informations relatives à la désinfection et au stockage et élabore ainsi une instruction d'autorisation de l'utilisation ou une instruction d'interdiction de l'utilisation de la sonde en fonction d'une analyse de la légitimité de l'utilisation de la sonde du point de vue hygiénique en fonction de ces informations. En outre, le croisement de toutes ces informations crée une liste d'attente, s'agrémentant tous les instants, des appareils médicaux nécessitant une désinfection. Cette liste d'attente permet aux personnes chargées de la désinfection de savoir quels appareils désinfecter en priorité et aux enceintes de stockage de refuser les appareils figurant toujours sur cette liste d'attente.

Ainsi, si le type de traitement pour lequel est utilisée la sonde requière un degré de désinfection particulier, l'unité de traitement vérifie si la sonde a été désinfectée au degré requis et autorise l'utilisation si c'est le cas, et interdit l'utilisation dans le cas contraire.

Si l'environnement dans lequel est utilisé la sonde (température, humidité, etc.) ne correspond pas à un environnement idéal d'utilisation de la sonde, l'unité de traitement interdit l'utilisation de la sonde.

D'autres critères d'autorisation et d'interdiction de la sonde peuvent être prévus et enregistrés dans l'unité de traitement de données D qui élabore l'instruction d'autorisation et d'interdiction de la sonde en fonction de ces critères et des informations transmises au cours des étapes mentionnées ci-dessus. Ces critères peuvent par exemple correspondre à des normes établies dans le domaine médical et sont enregistrés dans l'unité de traitement D qui vérifie si ces critères sont bien respectés préalablement à toute autorisation d'utilisation de la sonde. Toutes ces critères sont combinés afin d'autoriser ou d'interdire l'utilisation de la sonde.

A cet effet, le système de suivi est pourvu de moyens de stockages des informations acquises et traitées par le système d'acquisition et de traitement.

Un historique du suivi de l'utilisation de la sonde est également enregistré. De la sorte, après une utilisation de la sonde par un médecin, l'unité de traitement des données D peut élaborer des instructions de désinfection particulières (degré, type de désinfection) en fonction du type de traitement pour lequel la sonde a été utilisée, comme représenté par la flèche 7 de la figure 1.

De même, si la sonde a une durée de vie connue particulière le système de suivi peut déterminer si la sonde peut encore être utilisée ou si elle doit être remplacée en fonction de l'historique enregistré.

Ainsi, l'invention permet de garantir qu'une sonde particulière est toujours utilisée de façon légitime du point de vue de l'hygiène et d'éviter une utilisation dangereuse de cette sonde.

## Revendications

1. Procédé de suivi de l'utilisation d'un appareil médical du type sonde afin de déterminer si cet appareil peut être utilisé sans risque du point de vue hygiénique, ledit appareil comprenant des moyens d'identification dudit appareil, le procédé utilisant un système d'acquisition et de traitement d'informations comprenant des moyens d'acquisition des informations d'identification (Iᵢ) dudit appareil médical, des moyens d'acquisition d'informations relatives à la désinfection et à l'utilisation dudit appareil médical et des moyens d'association des informations d'identification et d'utilisation dudit appareil médical, ledit système comprenant un appareil indépendant et portable apte à communiquer avec un système de traitement d'informations, ledit appareil comprenant les moyens d'acquisition des informations d'identification (Iᵢ), les moyens d'acquisition d'informations d'utilisation relatives à l'utilisation dudit appareil médical et des moyens d'horodatage, le procédé étant **caractérisé en ce qu'**il comprend les étapes suivantes :
- lors de la désinfection (A) de l'appareil, enregistrement (1) par ledit appareil indépendant de la date de la désinfection (Iₕ) et des informations de désinfection (I_{d}) relatives au type et au degré de désinfection de l'appareil médical en relation avec les informations d'identification (Iᵢ) dudit appareil médical,
- lors de l'utilisation (C) de l'appareil médical sur un patient, enregistrement (5) par ledit appareil indépendant de la date et des informations d'utilisation relatives au type d'utilisation de l'appareil médical et au patient en relation avec les informations d'identification (Iᵢ) dudit appareil médical,
- séquençage par le système d'acquisition et de traitement des informations de désinfection (I_{d}) et des informations d'utilisation sur un patient en relation avec les informations d'identification (Iᵢ) de l'appareil médical,
- préalablement à chaque utilisation, élaboration par le système d'acquisition et de traitement d'une instruction d'autorisation ou d'interdiction (6) de l'utilisation de l'appareil médical à partir du séquençage, l'utilisation étant autorisée si, suite à une utilisation particulière, une désinfection adaptée à cette utilisation a eu lieu, et l'utilisation étant interdite dans le cas contraire.

2. Procédé de suivi selon la revendication 1, **caractérisé en ce qu'**il comprend en outre une étape d'enregistrement (2) d'une information relative au temps de stockage (B) de l'appareil médical par ledit appareil indépendant, ladite information de stockage étant séquencée avec les informations de désinfection et d'utilisation, le système d'acquisition et de traitement élaborant une instruction de désinfection si le temps de stockage dépasse un seuil prédéterminé et une instruction d'interdiction d'utilisation de l'appareil médical si cette désinfection n'est pas effectuée.

3. Procédé selon la revendication 1 ou 2, **caractérisé en ce que** l'enregistrement des informations de désinfection relatives au type et au degré de la désinfection comprend l'enregistrement du principe actif utilisé pour la désinfection, le temps d'exposition à ce principe actif de l'appareil médical, la température de désinfection et/ou le dosage du principe actif.

4. Procédé selon la revendication 3, **caractérisé en ce que** le système d'acquisition et de traitement élabore une information de suffisance ou d'insuffisance de la désinfection en fonction des informations de désinfection relatives au type et au degré de désinfection, le système élaborant une instruction d'interdiction d'utilisation de l'appareil médical si l'information d'insuffisance de la désinfection est élaborée.

5. Procédé selon l'une quelconque des revendications 1 à 4, **caractérisé en ce qu'**au cours de la désinfection (A) de l'appareil médical, un enregistrement de l'identité de l'opérateur effectuant la désinfection est effectué.

6. Procédé selon l'une quelconque des revendications 1 à 5, **caractérisé en ce que** l'enregistrement des informations d'utilisation relatives au type d'utilisation de l'appareil médical comprend l'enregistrement de l'identité du médecin traitant, de l'identité du patient, du type de traitement pour lequel l'appareil est utilisé, du type de pathologie du patient, de la durée d'utilisation de l'appareil, des informations sur l'environnement dans lequel est utilisé l'appareil et/ou des informations sur le système dans lequel est utilisé l'appareil.

7. Procédé selon l'une quelconque des revendications 1 à 6, **caractérisé en ce que** le système d'acquisition et de traitement enregistre un historique du suivi de l'utilisation de l'appareil médical et de sa légitimité d'utilisation.

8. Système de suivi de l'utilisation d'un appareil médical du type sonde permettant de mettre en oeuvre le procédé selon l'une quelconque des revendications 1 à 7, ledit système étant **caractérisé en ce qu'**il comprend des moyens d'identification associés à l'appareil médical, un appareil indépendant et portable apte à communiquer avec un système de traitement d'informations et comprenant des moyens d'acquisition des informations d'identification (Iᵢ) dudit appareil médical, des moyens d'acquisition d'informations d'utilisation relatives à l'utilisation dudit appareil médical, des moyens d'horodatage et des moyens d'association des informations d'identification (Iᵢ), d'utilisation dudit appareil médical et d'horodatage.

9. Système de suivi selon la revendication 8 , **caractérisé en ce que** les moyens d'identification associés à l'appareil médical comprennent une puce RFID fixée à demeure sur ledit appareil.

10. Système de suivi selon la revendication 8 ou 9, **caractérisé en ce que** les moyens d'acquisition des informations d'identification (Iᵢ) de l'appareil médical du système d'acquisition et de traitement comprennent des moyens de lecture à distance d'une puce RFID.

11. Système de suivi selon l'une des revendications 8 à 10, **caractérisé en ce qu'**il comprend des moyens de mesure du degré de désinfection fourni par une enceinte de désinfection.

12. Système de suivi selon l'une quelconque des revendications 8 à 11, **caractérisé en ce qu'**il comprend des moyens d'entrée d'informations relatives à l'utilisation de l'appareil médical.

13. Système de suivi selon l'une quelconque des revendications 8 à 12, **caractérisé en ce qu'**il comprend des moyens de stockages des informations acquises et traitées par le système d'acquisition et de traitement.

## Patentansprüche

1. Verfahren zur Überwachung der Verwendung eines medizinischen Geräts des Typs Sonde, um zu ermitteln, ob dieses Gerät im Hinblick auf Hygiene ohne Risiko verwendet werden kann, wobei das Gerät Mittel zur Identifikation des Geräts aufweist, wobei das Verfahren ein System zur Erlangung und zur Verarbeitung von Informationen verwendet, welches aufweist Mittel zur Erlangung von Informationen zur Identifikation (Iᵢ) des medizinischen Geräts, Mittel zur Erlangung von Informationen bezüglich der Desinfektion und der Verwendung des medizinischen Geräts und Mittel zur Assoziation der Informationen zur Identifikation und der Verwendung des medizinischen Geräts, wobei das System aufweist ein unabhängiges und tragbares Gerät, das in der Lage ist, mit einem Informationsverarbeitungssystem zu kommunizieren, wobei das Gerät aufweist die Mittel zur Erlangung der Informationen zur Identifikation(Iᵢ), die Mittel zur Erlangung von Informationen zur Verwendung bezüglich der Verwendung des medizinischen Geräts und Zeiterfassungs-Mittel, wobei das Verfahren **dadurch gekennzeichnet ist, dass** es die nachfolgenden Schritte aufweist:
- bei der Desinfektion (A) des Geräts, Eintragung (1) mittels des unabhängigen Geräts des Zeitpunkts der Desinfektion (Iₕ) und von Informationen zur Desinfektion (I_{d}) bezüglich des Typs und des Grads der Desinfektion des medizinischen Geräts in Relation zu den Informationen zur Identifikation (Iᵢ) des medizinischen Geräts,
- bei der Verwendung (C) des medizinischen Geräts an einem Patienten, Eintragung (5) mittels des unabhängigen Geräts des Zeitpunkts und der Informationen zur Verwendung bezüglich des Typs der Verwendung des medizinischen Geräts und des Patienten in Relation zu den Informationen zur Identifikation (Iᵢ) des medizinischen Geräts,
- Aufschlüsselung mittels des Systems zur Erlangung und Verarbeitung der Informationen zur Desinfektion (Is) und der Informationen zur Verwendung an einem Patienten in Relation zu den Informationen zur Identifikation (Iᵢ) des medizinischen Geräts,
- vor jeder Verwendung, Ausarbeitung mittels des Systems zur Erlangung und zur Verarbeitung einer Anweisung zur Autorisierung oder Unterbindung (6) der Verwendung des medizinischen Geräts ausgehend von der Aufschlüsselung, wobei die Verwendung autorisiert wird, wenn auf eine bestimmte Verwendung folgend eine an diese Verwendung angepasste Desinfektion stattgefunden hat, und die Verwendung im gegenteiligen Falle unterbunden wird.

2. Verfahren zur Überwachung gemäß Anspruch 1, **dadurch gekennzeichnet, dass** es ferner aufweist einen Schritt der Eintragung (2) einer Information bezüglich der Lagerungszeit (B) des medizinischen Geräts mittels des unabhängigen Geräts, wobei die Information der Lagerung aufgeschlüsselt wird mit den Information zur Desinfektion und der Verwendung, wobei das System zur Erlangung und zur Verarbeitung eine Anweisung zur Desinfektion, wenn die Lagerungszeit eine vorbestimmte Grenze passiert, und eine Anweisung der Unterbindung der Verwendung des medizinischen Geräts ausarbeitet, wenn diese Desinfektion nicht durchgeführt wird.

3. Verfahren gemäß Anspruch 1 oder 2, **dadurch gekennzeichnet, dass** die Eintragung von Informationen zur Desinfektion bezüglich des Typs und des Grads der Desinfektion aufweist die Eintragung des Wirkstoffs, der für die Desinfektion verwendet wird, der Zeit der Beaufschlagung des medizinischen Geräts mit dem Wirkstoff, der Desinfektionstemperatur und/oder der Dosierung des Wirkstoffs.

4. Verfahren gemäß Anspruch 3, **dadurch gekennzeichnet, dass** das System zur Erlangung und zur Verarbeitung eine Information des Ausreichens oder des Nicht-Ausreichens der Desinfektion in Abhängigkeit von den Informationen zur Desinfektion bezüglich des Typs und des Grads der Desinfektion ausarbeitet, wobei das System eine Anweisung zur Unterbindung der Verwendung des medizinischen Geräts ausarbeitet, wenn die Information des Nicht-Ausreichens der Desinfektion ausgearbeitet wird.

5. Verfahren gemäß irgendeinem der Ansprüche 1 bis 4, **dadurch gekennzeichnet**, das im Laufe der Desinfektion (A) des medizinischen Geräts eine Eintragung der Identität des Operateurs vorgenommen wird, der die Desinfektion vornimmt.

6. Verfahren gemäß irgendeinem der Ansprüche 1 bis 5, **dadurch gekennzeichnet, dass** die Eintragung der Informationen zur Verwendung bezüglich des Typs der Verwendung des medizinischen Geräts aufweist die Eintragung der Identität des behandelnden Mediziners, der Identität des Patienten, des Typs der Behandlung, für die das Gerät verwendet wird, des Typs der Pathologie des Patienten, der Dauer der Verwendung des Geräts, von Informationen über die Umgebung, in welcher das Gerät verwendet wird, und/oder von Informationen über das System, in welchem das Gerät verwendet wird.

7. Verfahren gemäß irgendeinem der Ansprüche 1 bis 6, **dadurch gekennzeichnet, dass** das System zur Erlangung und zur Verarbeitung eine Historie der Überwachung der Verwendung des medizinischen Geräts und seiner Legitimität zur Verwendung einträgt.

8. System zur Überwachung der Verwendung eines medizinischen Geräts des Typs Sonde, welches es erlaubt, das Verfahren gemäß irgendeinem der Ansprüche 1 bis 7 durchzuführen, wobei das System **dadurch gekennzeichnet ist, dass** es aufweist Mittel zur Identifikation, die mit dem medizinischen Gerät assoziiert sind, ein unabhängiges und tragbares Gerät, das in der Lage ist, mit einem Informationsverarbeitungssystem zu kommunizieren, und das aufweist Mittel zur Erlangung von Informationen zur Identifikation (Iᵢ) des medizinischen Geräts, Mittel zur Erlangung von Informationen zur Verwendung (Ii) bezüglich der Verwendung des medizinischen Geräts, Zeiterfassungs-Mittel und Mittel zur Assoziation der Informationen zur Identifikation (Ii), zur Verwendung des medizinischen Geräts und zur Zeiterfassung.

9. System zur Überwachung gemäß Anspruch 8, **dadurch gekennzeichnet, dass** die Mittel zur Identifikation, die mit dem medizinischen Gerät assoziiert sind, aufweisen einen RFID-Chip, der dauerhaft an dem Gerät fixiert ist.

10. System zur Überwachung gemäß Anspruch 8 oder 9, **dadurch gekennzeichnet, dass** die Mittel zur Erlangung der Informationen zur Identifikation (Iᵢ) des medizinischen Geräts des Systems zur Erlangung und zur Verarbeitung aufweisen Mittel zum Lesen eines RFID-Chips auf Distanz.

11. System zur Überwachung gemäß einem der Ansprüche 8 bis 10, **dadurch gekennzeichnet, dass** es aufweist Mittel zur Messung des Grads der Desinfektion, der von einem Desinfektionsbereich geliefert wird.

12. System zur Überwachung gemäß irgendeinem der Ansprüche 8 bis 11, **dadurch gekennzeichnet, dass** es aufweist Mittel zum Zugang zu Information bezüglich der Verwendung des medizinischen Geräts.

13. System zur Überwachung gemäß irgendeinem der Ansprüche 8 bis 12, **dadurch gekennzeichnet, dass** es aufweist Mittel zur Speicherung der Informationen, die mittels des Systems zur Erlangung und Verarbeitung erlangt und verarbeitet wurden.

## Claims

1. Method for tracking the use of a medical apparatus of the probe type in order to determine whether said apparatus can be safely used from a hygienic standpoint, wherein said apparatus comprises an apparatus identifier, wherein the method uses an information acquisition and processing system comprising means for acquiring identification information (Ii) of said medical apparatus, the means for acquiring disinfection and use information of said medical apparatus said system comprising an independent portable device able to communicate with an information processing system, said device comprising the means for acquiring disinfection and use information of said medical apparatus and means for associating the identification information and the use information of said medical apparatus, and means for acquiring the date and time, the method being **characterized in that** it comprises the following steps:
- during the disinfection (A) of the apparatus, recording (1) by said independent device of the disinfection date (Ih) and disinfection information (Id) regarding the type and degree of disinfection of the medical apparatus in conjonction with the identification information (li) of said medical apparatus,
- during the use (C) of the medical apparatus on a patient, recording (5) by said independent apparatus of the date and of the use information regarding the type of use of the medical apparatus and the patient in conjunction with the identification information (Ii) of said medical apparatus,
- sequencing by the information acquisition and processing system of the disinfection information (Id) and of the use information on a patient in conjonction with the identification information of the medical apparatus,
- before each use, elaborating by the information acquisition and processing system an authorization or a prohibition instruction (6) for the use of the medical apparatus based on the sequencing, the use being authorized if, following a particular use, a disinfection adapted to this use has occurred, and the use being prohibited if not.

2. Tracking method according to claim 1, **characterized in that** it additionally comprises a stage in which information regarding the apparatus storage time is recorded by the independent device, wherein said storage information is sequenced in with the disinfection and use information, and the information acquisition and processing system produces a disinfection order if the storage time exceeds a predetermined threshold and an order prohibiting the use of the apparatus if it is not disinfected.

3. Method according to claim 1, **characterized in that** recording the disinfection information about the type and degree of disinfection includes recording the active principle used for disinfection, the length of time the apparatus was exposed to this active principle, the disinfection temperature and/or the active principle dosage.

4. Method according to claim 3, **characterized in that** the information acquisition and processing system produces a notice of adequate or inadequate disinfection based on the disinfection information about the type and degree of disinfection, wherein if a notice of inadequate disinfection is produced, the system prohibits the use of the apparatus.

5. Method according to any of one claims 1 to 4, **characterized in that**, while the apparatus is being disinfected, the identity of the operator performing the disinfection is recorded.

6. Method according to any one of claims 1 to 5, **characterized in that** recording the use information regarding the type of use of the apparatus includes recording the identity of the attending physician, the identity of the patient, the type of treatment for which the apparatus is used, the type of pathology of the patient, the length of time the apparatus is used, the information on the environment in which the apparatus is used and/or the information on the system in which the apparatus is used.

7. Method according to any one of claims 1 to 6, **characterized in that** the information acquisition and processing system records a history tracking the use of the medical apparatus and the legitimacy of its use.

8. System for tracking the use of a medical apparatus of the probe type that makes it possible to implement the method according to any one of claims 1 to 7, wherein said system comprises, an information acquisition and processing system comprising an independent and portable device able to communicate with an information processing system and that acquires information identifying said medical apparatus, acquires use information regarding the use of said medical apparatus, records the date and time, and associates identification and use information for said medical apparatus with date and time information.

9. Tracking system according to claim 8, **characterized in that** the identifier associated with the medical apparatus comprises a RFID chip permanently fixed on said apparatus.

10. Tracking system according to claim 8 or 9, **characterized in that** the identity acquisition component comprises a RFID chip scanner.

11. Tracking system according to any one of claims 8 to 10, **characterized in that** it comprises means for measuring the degree of disinfection provided by a disinfection enclosure.

12. Tracking system according to any one of claims 8 to 11, **characterized in that** it comprises means for imputing information relative to the use of the medical apparatus.

13. Tracking system according to any one of claims 8 to 12, **characterized in that** it comprises means for storing the acquired and processed system by the acquisition and processing system.
